# EUROPÄISCHE PATENTSCHRIFT

(11) **EP 3 439 566 B1**
(45) Veröffentlichungstag und Bekanntmachung des Hinweises auf die Patenterteilung: **08.05.2024**
(21) Anmeldenummer: 17716162.7
(22) Anmeldetag: 04.04.2017
(51) Int. Cl.: A61B 17/88, A61B 17/84

(54) **KNOCHENIMPLANTAT MIT VERSATZ IM INJEKTIONSKANAL SOWIE IMPLANTATIONSWERKZEUG HIERFÜR**
BONE IMPLANT HAVING AN OFFSET IN THE INJECTION CHANNEL AND IMPLANTATION TOOL THEREFOR
IMPLANT OSSEUX AVEC DÉCALAGE DANS LE CANAL D'INJECTION ET OUTIL D'IMPLANTATION CORRESPONDANT

(30) Priorität: 06.04.2016 DE 102016106308
(43) Veröffentlichungstag der Anmeldung: 13.02.2019
(73) Patentinhaber: Aesculap AG, 78532 Tuttlingen (DE)
(72) Erfinder: ACKERMANN, Janina, 78532 Tuttlingen (DE); MARX, Irene, 78647 Trossingen (DE); LINDNER, Stephan, 78573 Wurmlingen (DE); KRÜGER, Sven, 78647 Trossingen (DE); GNOTH, Barbara, 78194 Immendingen (DE)
(74) Vertreter: Winter, Brandl - Partnerschaft mbB
(86) Internationale Anmeldenummer: PCT/EP2017/057966
(87) Internationale Veröffentlichungsnummer: WO 2017/174569

(56) Entgegenhaltungen:
- US-A1- 2004 225 292
- US-A1- 2013 072 984
- US-A1- 2014 194 886
- US-B1- 7 338 493

## Beschreibung

Die vorliegende Erfindung betrifft ein Knochenimplantat mit einem Implantatschaft zum Anordnen in einem Knochen und einem proximal am Implantatschaft angeordneten Implantatkopf mit einer Werkzeugaufnahme für ein Implantationswerkzeug, wobei der Implantatschaft eine Längsachse A besitzt und in dem Implantatschaft ein durchgehender Injektionskanal zum Injizieren eines Knochenverbindungsmaterials ausgebildet ist, der auf Seiten der Werkzeugaufnahme eine proximale Einlassöffnung für das Knochenverbindungsmaterial aufweist. Die Erfindung betrifft des Weiteren ein Implantationsinstrument für ein Knochenimplantat nach einem der vorstehenden Ansprüche, das eine Injektionskanüle mit einem Innenkanal mit einer Längsachse B zum Injizieren von Knochenverbindungsmaterial aufweist. Sie betrifft schließlich ein Implantationssystem mit einem Knochenimplantat nach der Erfindung sowie einem Implantationsinstrument nach der Erfindung.

Knochenimplantate, wie zum Beispiel Gelenksprothesen, Knochen- oder Pedikelschrauben sind aus dem Stand der Technik bekannt. Sie werden in einen Knochen eingebracht und dort befestigt. Ihre Befestigung kann zum Beispiel durch Einschrauben und alternativ oder zusätzlich mittels einer Knochenverbindungsmasse oder eines Knochenverbindungsmaterial, auch als Knochenzement bezeichnet, erfolgen. Ein Knochenimplantat weist zu diesem Zweck zumindest einen Kanal auf, auch als Injektionskanal oder Zementkanal bezeichnet, durch den die Knochenverbindungsmasse /-material bei der Implantierung in der gewünschten Weise und an die gewünschte Lokalität eingebracht und platziert werden kann.

Beim Zementieren von Knochenimplantaten besteht das Problem, dass es beim Entfernen einer zum Einbringen von Knochenzement in einen dafür vorgesehenen Kanal des Implantats genutzten Kanüle zu einem Austritt von ggf. ausgehärtetem Knochenzement kommen kann, ähnlich einem Kleberfaden, welcher sich nach Beenden eines Klebvorgangs nicht ohne Weiteres von der Kleberflasche löst. Derartige Zementbestandteile können in nachteiliger Weise eine bestimmungsgemäße Verwendung und/oder Manipulation/Einrichtung des Implantats einschränken, gar verhindern, oder sich lösen und eine Gefahr für den Patienten darstellen. Dieser nachteilige Austritt von Knochenzement kann sowohl aus dem Implantat als auch aus der Kanüle erfolgen.

Pedikelschrauben dienen beispielsweise zur dorsalen Stabilisierung der Wirbelsäule mittels transpedikulärer Verschraubung. Dabei werden Pedikelschrauben in den Pedikeln jeweils benachbarter Wirbel platziert, worauf eine winkelstabile Verbindung zwischen den jeweils axial übereinander angeordneten Pedikelschrauben und einem sich axial erstreckenden Längsträger oder Steg geschaffen wird. Der Längsträger wird dabei in eine schraubenkopfseitige Aufnahmehülse, die sogenannte Tulpe, eingebracht und dort verspannt. Auf diese Weise bilden die Pedikelschrauben und der Längsträger ein Wirbel-Stabilisierungssystem. Im Falle einer Zementierung von fenestrierten Pedikelschrauben kann es zum Beispiel beim Abziehen einer Augmentationskanüle dazu kommen, dass ein Teil des ausgehärteten Zements aus der Pedikelschraube und/oder aus der Augmentationskanüle herausgezogen wird und derart bricht, dass eine Zementsäule aus der Pedikelschraube herausragt. Diese kann weitere Operationsschritte erschweren oder sogar unmöglich machen. Ein unbemerktes Lösen von Teilen oder Abschnitten einer solchen Zementsäule stellt eine nicht unwesentliche Gefahr für das Wohlbefinden, die Gesundheit oder das Leben des Patienten dar.

Aus der DE 10 2013 109 895 A1 sind ein Implantat, z.B. in Form einer Polyaxialschraube mit einen Schraubenschaft und einem darin ausgebildeten Längskanal, sowie ein medizinisches Instrument zu dessen Implantierung bekannt, wobei das Instrument einen Werkzeugabschnitt mit einem ersten Werkzeugelement zum in Eingriff-Bringen mit einem korrespondierenden zweiten Werkzeugelement in dem Implantat aufweist, wobei am oder im Instrument ein Knochenzementbehälter ausgebildet oder angeordnet ist. Ein distales Ende des Schraubenschafts ist offen und steht mit dem Längskanal in Fluidverbindung. Das proximale Ende des Schraubenschafts ist in Form eines kugeligen Kopfes ausgebildet, auf dem eine Hülse gelenkig gelagert ist. Das proximale Ende des Längskanals mündet innerhalb des Kugelkopfs. Das Instrument und das Implantat sind mittels der beiden Werkzeugelemente in Eingriff zu bringen, wodurch eine Fluidverbindung zwischen dem Knochenzementbehälter und dem Längskanal erstellt wird.

Aus der DE 10 2008 024 440 A1 ist ein ähnliches Implantat mit einer Werkzeugaufnahme zum Verbinden mit einem Implantationswerkzeug und mit einer Verbindungseinrichtung zum Verbinden des Implantats mit einer Injektionskanüle zum Injizieren eines Knochenverbindungsmaterials bekannt, wobei die Verbindungseinrichtung einen Innengewindeabschnitt umfasst, der in oder an der Werkzeugaufnahme ausgebildet ist.

Aus der DE 20 2012 008 715 U1 ist eine Pedikelschraube bekannt, die kanüliert und mit seitlichen Bohrungen oder Ausfräsungen versehen wurde und bei der ein vollständiger oder teilweiser Verschluss der Kanülierung am distalen Ende der Schraube ermöglicht wurde.

Aus der CN 2032 52718 U ist eine Pedikelschraube bekannt, die mit einem Innenkanal und Durchtrittöffnungen im Schraubenschaft und im Gewindeabschnitt versehen ist. In einem Kopf der Pedikelschraube ist ein Innengewinde zum Verbinden der Schraube mit einer Injektionskanüle zum Applizieren von Knochenzement ausgebildet.

Ferner ist aus US 2004 225 292 A1 ein Knochenanker mit einem schraubenartigen Knocheneingriffsabschnitt und einem länglichen Führungsabschnitt zur Führung einer Vorrichtung zum Eingriff mit dem Knocheneingriffsabschnitt bekannt. In einer Ausführungsform umfasst der Knocheneingriffsabschnitt einen Kanülendurchgang. Nach einer Ausführungsform kann ein proximaler Abschnitt des Knocheneingriffsabschnitts mit einem Innengewinde zur Befestigung des Führungsabschnitts versehen sein und kann ferner ein weiteres Innengewinde im proximalen Kopf des Knocheneingriffsabschnitts definiert sein, welches zum Eingriff mit der Vorrichtung geeignet ist. Die US 2014/194886 A1 offenbart ein weiteres Beispiel für ein Implantationssystem mit einem Implantations- und Injektionsinstrument für und mit einem Knochenimplantat in Form einer Pedikelschraube.

Darüber hinaus offenbart US 7 338 493 B1 einen Knochenschraubenanker, der in der Lage ist, eine Fixierungsvorrichtung sicher an einem schwachen oder brüchigen Knochen zu befestigen. Der Anker hat einen proximalen Bereich, der eine Aufnahme für einen Treiber aufweist, der den Anker in eine Bohrung oder ein Loch im Knochen treibt. Der Anker hat eine Innenbohrung mit Innengewinde zur Aufnahme einer Knochenschraube, die zur Befestigung einer Fixierungsvorrichtung am Anker dient. Knochenzement kann in die Bohrung eingebracht werden.

Der vorstehend beschriebene Stand der Technik adressiert nur die Dichtheit zwischen der Zementkanüle und der Knochenschraube. Zu diesen Zweck sind Gewinde sowohl in der Zementkanüle als auch in der Knochenschraube bekannt. Der Bereich, in dem der Innenkanal der Zementkanüle und der Injektionskanal der Knochenschraube mit dem Knochenzement in Kontakt kommen, ist bei bekannten Systemen stets glatt und ohne Hinterschnitt ausgebildet. Dadurch bedingt, kann es bei einem Trennen von Zementinjektor und Implantat in nachteiliger Weise zu einem Herausziehen von ggf. ausgehärteten Zementbestandteilen aus dem Instrument und/oder dem Längskanal des Implantats kommen.

Ausgehend vom vorgenannten Stand der Technik liegt der Erfindung die Aufgabe zugrunde, ein Knochenimplantat und ein Knochenimplantationssystem zu schaffen, dass die vorstehenden Nachteile nicht aufweist. Insbesondere soll ein Austreten oder gar Ablösen von Knochenverbindungsmaterial aus dem Implantat sowie aus dem zum Applizieren genutzten medizinischen Instrument sicher vermieden werden. Das Knochenimplantat und das Knochenimplantationssystem sollen einfach und sicher handzuhaben sowie kostengünstig sein.

Die Aufgabe wird gelöst durch ein Implantationsinstrument nach Anspruch 1, ein Implantationssystem nach Anspruch 7 und/ oder ein Knochenimplantat in Form einer Pedikelschraube nach Anspruch 14.

Genauer ausgedrückt, wird die Aufgabe gelöst durch ein Implantationsinstrument für ein Knochenimplantat vorzugsweise in Form einer Pedikelschraube, aufweisend eine Injektionskanüle mit einem Innenkanal mit einer Längsachse B zum Injizieren von Knochenverbindungsmaterial, wobei der Innenkanal eine distale Auslassöffnung für Knochenverbindungsmaterial aufweist und dort einen vorbestimmten Innenkanaldurchmesser hat, wobei die den Innenkanal umgebende Wand der Injektionskanüle ausgehend von der distalen Auslassöffnung einen zur Längsachse B radialen Versatz aufweist, vorzugsweise ein in die Wand des Innenkanals eingebrachtes Gewinde oder eine in die Wand des Innenkanals eingebrachte Nut, aufweist, der sich gegenüber dem sowohl distal als auch proximal zum Versatz ausgebildeten vorbestimmten Innenkanaldurchmesser radial nach außen erstreckt, wobei der radiale Versatz zumindest in einem Bereich der Wand ausgebildet ist, der von der distalen Auslassöffnung zwischen ca. 0,5 mm bis ca. 10 mm beabstandet ist.

In anderen Worten ausgedrückt, ist der radiale Versatz in einem Bereich der Wand ausgebildet, der sich betreffend die Injektionskanüle in der Nähe der distalen Auslassöffnung oder auf Seiten der distalen Auslassöffnung befindet. Man kann auch sagen, dass der radiale Versatz in einem an die distale Auslassöffnung angrenzenden Abschnitt der Kanalwand ausgebildet ist. Der radiale Versatz ist von der distalen Auslassöffnung wenigstens ca. 0,5 mm und maximal ca. 10 mm beabstandet, und kann dazwischen beliebige Werte annehmen. Er kann nach einer Ausführungsform zwischen ca. 1 mm bis ca. 7,5 mm, nach einer weiteren Form zwischen ca. 1,5 mm bis ca. 5 mm und nach einer weiteren Ausführungsform zwischen ca. 2 mm bis ca. 3 mm von der distalen Auslassöffnung beabstandet sein.

Alternativ oder zusätzlich wird die Aufgabe gelöst durch ein Implantationssystem mit einem Implantationsinstrument für ein Knochenimplantat vorzugsweise in Form einer Pedikelschraube, aufweisend eine Injektionskanüle mit einem Innenkanal mit einer Längsachse B zum Injizieren von Knochenverbindungsmaterial, wobei der Innenkanal eine distale Auslassöffnung für Knochenverbindungsmaterial aufweist und dort einen vorbestimmten Innenkanaldurchmesser hat, wobei die den Innenkanal umgebende Wand der Injektionskanüle ausgehend von der distalen Auslassöffnung einen zur Längsachse B radialen Versatz aufweist, vorzugsweise ein in die Wand des Innenkanals eingebrachtes Gewinde oder eine in die Wand des Innenkanals eingebrachte Nut aufweist, der sich gegenüber dem vorbestimmten Innenkanaldurchmesser, der sich sowohl distal als auch proximal zum Versatz ausbildet, radial nach außen erstreckt, vorzugsweise einem vorstehend beschriebenen Implantationsinstrument, sowie einem Knochenimplantat mit einem distalen Implantatschaft, der zum Versenken in einem Knochen ausgebildet ist, und einem proximalen Implantatkopf mit einer Werkzeugaufnahme für ein Implantationswerkzeug, von der sich ein Injektionskanal zum Injizieren eines Knochenverbindungsmaterials in den Implantatschaft erstreckt und dort einen vorbestimmten Injektionskanaldurchmesser hat, wobei die den Injektionskanal umgebende Wand ausgehend von der Werkzeugaufnahme einen Versatz, vorzugsweise ein in die Wand des Injektionskanals eingebrachtes Gewinde oder eine in die Wand des Injektionskanals eingebrachte Nut, aufweist, der sich gegenüber dem vorbestimmten Injektionskanaldurchmesser proximal und distal zum Versatz radial nach außen erstreckt.

Alternativ oder zusätzlich wird die Aufgabe gelöst durch ein Knochenimplantat in Form einer Pedikelschraube für ein vorstehend beschriebenes Implantationssystem, mit einem distalen Implantatschaft, der zum Versenken in einem Knochen ausgebildet ist, und einem proximalen Implantatkopf mit einer Werkzeugaufnahme für ein Implantationswerkzeug, von der sich ein Injektionskanal zum Injizieren eines Knochenverbindungsmaterials in den Implantatschaft erstreckt und dort einen vorbestimmten Injektionskanaldurchmesser hat, wobei die den Injektionskanal umgebende Wand ausgehend von der Werkzeugaufnahme einen Versatz in Form einer in die Wand des Injektionskanals eingebrachten singulären Nut aufweist, die sich gegenüber dem vorbestimmten Injektionskanaldurchmesser, der sich proximal und distal zum Versatz ausbildet, radial nach außen erstreckt, wobei die singuläre Nut durch einen einzigen, in die Wand des Injektionskanals eingebrachten Hinterschnitt ausgebildet ist.

Zusammengefasst hat bei dem Knochenimplantat nach Anspruch 14 die den Injektionskanal umgebende Wand des Implantschafts auf Seiten der proximalen Einlassöffnung einen zur Längsachse A radialen Versatz. Ein Implantationsinstrument nach Anspruch 1 für ein Knochenimplantat hat eine Injektionskanüle mit einem Innenkanal mit einer Längsachse B zum Injizieren von Knochenverbindungsmaterial, wobei der Innenkanal eine distale Austrittöffnung für Knochenverbindungsmaterial aufweist, wobei die den Innenkanal umgebende Wand der Injektionskanüle auf Seiten der distalen Austrittsöffnung einen zur Längsachse B radialen Versatz aufweist. Ein Implantationssystem nach Anspruch 7 hat ein Knochenimplantat sowie ein Implantationsinstrument nach der Erfindung.

Nochmals zusammengefasst wird ein Knochenimplantat vorgeschlagen, mit einem distalen Implantatschaft, der zum Versenken in einem Knochen ausgebildet ist, und einem proximalen Implantatkopf mit einer Werkzeugaufnahme für ein Implantationswerkzeug, von der sich ein Injektionskanal zum Injizieren eines Knochenverbindungsmaterials in den Implantatschaft erstreckt und dort einen vorbestimmten Injektionskanaldurchmesser hat, wobei die den Injektionskanal umgebende Wand ausgehend von der Werkzeugaufnahme einen Versatz, vorzugsweise ein in die Wand des Injektionskanals eingebrachtes Gewinde oder eine in die Wand des Injektionskanals eingebrachte Nut aufweist, der sich gegenüber dem vorbestimmten Injektionskanaldurchmesser proximal und distal zum Versatz radial nach außen erstreckt, zudem wird ein Implantationsinstrument für ein erfindungsgemäßes Knochenimplantat vorgeschlagen, aufweisend eine Injektionskanüle mit einem Innenkanal mit einer Längsachse B zum Injizieren von Knochenverbindungsmaterial, wobei der Innenkanal eine distale Auslassöffnung für Knochenverbindungsmaterial aufweist und dort einen vorbestimmten Innenkanaldurchmesser hat, wobei die den Innenkanal umgebende Wand der Injektionskanüle ausgehend von der distalen Auslassöffnung einen zur Längsachse B radialen Versatz aufweist, vorzugsweise ein in die Wand des Innenkanals eingebrachtes Gewinde oder eine in die Wand des Innenkanals eingebrachte Nut aufweist, der sich gegenüber dem vorbestimmten Innenkanaldurchmesser distal und proximal zum Versatz radial nach außen erstreckt, und zudem wird ein Implantationssystem mit einem erfindungsgemäßen Knochenimplantat und einem erfindungsgemäßen Implantationsinstrument vorgeschlagen.

Funktional ausgedrückt offenbart die Erfindung an einem proximalen Ende eines Knochenimplantats respektive am distalen Ende eines Implantationswerkzeugs ein Haftreibungserhöhungselement, welches mittels des vorstehend beschriebenen Versatzes, einer proximalen Bewegung des Knochenverbindungsmaterials, ausgelöst durch ein Entfernen des Implantationswerkzeugs durch den Operateur, entgegenwirkt und somit eine Sollbruchstelle für das Knochenverbindungsmaterial vor / während dessen vollständigen Aushärtens gewährleistet. Wie im weiteren Verlauf dieser Anmeldung deutlich wird, kann das Haftreibungserhöhungselement des Implantationsinstruments verschiedene Formen annehmen, wie etwa eine Stufe, eine Nut, ein Gewinde, ein Hinterschnitt etc.

Das Knochenimplantat nach der Erfindung ist insbesondere dazu vorgesehen und geeignet, mit den Implantatschaft in einer Knochenkavität angeordnet zu werden und dort einzementiert zu werden. Der Injektionskanal ist in dem Implantatschaft zumindest abschnittsweise durchgehend ausgebildet. Er bildet eine fluidische Verbindung zwischen der proximalen Einlassöffnung und zumindest einer im Bereich des Implantatschafts ausgebildeten Auslassöffnung für das Knochenverbindungsmaterial aus. Nach der der Erfindung zugrundeliegenden Idee bildet der radiale Versatz in der Injektionskanüle eine Haltestruktur für in dem Injektionskanal befindliches Knochenverbindungsmaterial aus. Alternativ oder zusätzlich kann der Versatz derart ausgebildet und dimensioniert sein, dass eine Sollbruchstelle in dem in dem Injektionskanal befindliches Knochenverbindungsmaterial ausgebildet wird, wobei der Abschnitt, in welchem das Knochenverbindungsmaterial aushärtet, und die Werkzeugaufnahme als voneinander getrennte Abschnitte ausgebildet sind. Die Werkzeugaufnahme, welche proximal angeordnet ist, und der Hinterschnitt/der radiale Versatz, welcher weiter distal angeordnet ist, erfüllen unterschiedliche Funktionen.

Wird ein Injektionsinstrument nach erfolgtem Einbringen von Knochenverbindungsmaterial von implantierten Implantat entkoppelt und entfernt, wird das in dem Injektionskanal vorliegende Verbindungsmaterial durch den Versatz gehalten, zumindest in proximale Richtung gehalten, kann nicht in proximale Richtung im Kanal relativbewegt werden und aus dem Kanal und dem Implantat austreten. Ein gleicher Effekt nur in entgegengesetzte Richtung wird hinsichtlich des Innenkanals der Injektionskanüle des Implantationssystems nach der Erfindung bewirkt: Bei einem Entfernen des Instruments nach erfolgtem Einbringen von Knochenverbindungsmaterial von implantierten Implantat wird das in dem Innenkanal der Injektionskanüle vorliegende Verbindungsmaterial durch dessen Versatz gehalten, zumindest in distale Richtung gehalten, kann nicht in distale Richtung im Innenkanal der Injektionskanüle relativbewegt werden und daraus austreten. Insgesamt wird so durch die Erfindung ein Austreten von Knochenzement, insbesondere von bereits teilweise oder vollständig gehärtetem Knochenzement, aus dem Implantat sowie aus dem zu dessen Implantierung genutzten medizinischen Instrument sicher vermieden.

Wie vorhergehend beschrieben dienen der radiale Versatz in der Injektionskanalwand des Implantats und der radiale Versatz in der Innenkanalwand des Instrumentes dazu, die Haftung des Knochenverbindungsmittels in Axialrichtung zu erhöhen. Zur Erfüllung dieser Funktion ist beispielsweise auch eine erhöhte Rauigkeit der Kanalwände denkbar.

Ein besonderer Vorteil der Erfindung ist, dass durch den radialen Versatz, der erfindungsgemäß jeweils im Injektionskanal sowie im Innenkanal der Injektionskanüle ausgebildet ist, eine definierte Sollbruchstelle für darin befindlichen Knochenzement gebildet werden kann. Es ist daher stets sichergestellt, dass die jeweils gewünschte Menge an Knochenverbindungsmaterial im Implantat verbleibt. Auch kann durch eine solche Sollbruchstelle ein Ausbrechen von separaten Partikeln oder ein Zerbröseln von teilgehärtetem Knochenzement, sowie ein Kontaminieren der Implantierungsumgebung durch derartige Partikel vermieden werden. Ein Ziel der Erfindung ist, dass der Zement durch den Versatz in der Knochenschraube und der Zementkanüle im Bereich zwischen den beiden Versätzen definiert abbrechen kann und abbricht.

Vorteilhafte Ausführungsformen der Erfindung sind in den Unteransprüchen beansprucht und werden nachfolgend näher erläutert. In der Beschreibung der Erfindung betrifft die Bezeichnung "Kanal" sowohl den Injektionskanal des Implantats als auch den Innenkanals der Injektionskanüle.

Nach einer Ausführungsform der Erfindung , im Hinblick auf ein erfindungsgemäßes medizinisches Implantierungsinstrument, ist der radiale Versatz durch in die Wand des Innenkanals eingebrachte Stufen, Schultern oder Querschnittsänderungen ausgebildet. Die Stufen, Schultern oder Querschnittsänderungen bilden innerhalb des Kanals eine Anlagestruktur für das Knochenverbindungsmaterial aus, die im Wesentlichen quer oder deutlich geneigt zur Längsachse A bzw. B ausgerichtet ist. Sie bildet daher eine Art Hindernis, das einer Bewegung von Knochenzement, insbesondere im ausgehärteten und teilgehärteten Zustand, in Richtung der jeweiligen Längsachse A bzw. B entgegensteht, ein Injizieren von nicht gehärtetem Knochenverbindungsmaterial aber ohne wesentliche Einschränkung ermöglicht.

Alternativ oder zusätzlich kann der Versatz im Implantat wie auch im Instrument in umfänglicher Richtung, also beim Implantat mit Bezug auf die Längsachse A und beim Instrument mit Bezug auf die Längsachse B, zumindest abschnittsweise umlaufend ausgebildet sein. Durch eine entsprechende Formgebung des Versatzes in umlaufender Richtung, zum Beispiel durch scharfkantige Unterbrechungen des Versatzes, kann im Knochenverbindungsmaterial eine Kerbwirkung hervorgerufen werden, die ein Versagen des Materials an der gewünschten Sollbruchstelle begünstigt. Ein besonders stabiles Halten des Zements in den jeweiligen Kanälen wird durch einen vollständig und ununterbrochen umlaufenden Versatz gefördert.

Eine Ausführungsform der Erfindung ist dadurch gekennzeichnet, dass der radiale Versatz durch ein in die Wand des des Innenkanals eingebrachtes Gewinde oder durch einen in die Wand des Injektionskanals bzw. des Innenkanals eingebrachten Hinterschnitt ausgebildet ist. Derartige Strukturen sind trotz der problematischen Zugänglichkeit und üblichen Dimensionieren der betreffenden Implantate einfach, unaufwändig und kostengünstig herstellbar.

Eine weitere Ausführungsform der Erfindung ist dadurch gekennzeichnet, dass der radiale Versatz in einem Bereich der Wand ausgebildet ist, der sich (betreffend das Implantat) in der Nähe der proximalen Einlassöffnung oder auf Seiten der proximalen Einlassöffnung befindet. Man kann auch sagen, dass der radiale Versatz in einem an die proximale Einlassöffnung angrenzenden Abschnitt der Kanalwand ausgebildet ist. Der radiale Versatz kann insbesondere von der proximalen Einlassöffnung wenigstens ca. 0,5 mm und maximal ca. 10 mm beabstandet sein und dazwischen beliebige Werte annehmen. Er kann nach einer Ausführungsform zwischen ca. 1 mm bis ca. 7,5 mm, nach einer weiteren Form zwischen ca. 1,5 mm bis ca. 5 mm und nach einer weiteren Ausführungsform zwischen ca. 2 mm bis ca. 3 mm von der proximalen Einlassöffnung bzw. der distalen Auslassöffnung beabstandet sein.

Eine weitere Ausführungsform der Erfindung ist dadurch gekennzeichnet, dass der Versatz des Injektionskanals in radialer Richtung mit Bezug auf die Längsachse A bzw. der Versatz des Innenkanals mit Bezug auf die Längsachse B zwischen ca. 0,1 mm bis 3 mm, vorzugsweise zwischen ca. 0,2 mm bis 2 mm und besonders bevorzugt zwischen ca. 0,3 mm bis ca. 1,5 mm beträgt. In diesen Bereichen besteht der Vorteil, dass nicht ausgehärtetes Knochenverbindungsmaterial noch ohne Einschränkung oder zumindest nur unter unwesentlicher Einschränkung durch den jeweiligen Kanal injizierbar ist, dass aber teilweise oder vollständig ausgehärtetes Knochenverbindungsmaterial an Bewegungen in axialer Richtung sicher gehindert wird und gehalten wird.

Nach einer Ausführungsform der Erfindung ist vorgesehen, dass das Knochenimplantat eine Knochenschraube ist, insbesondere eine Pedikelschraube. Diese kann einen insbesondere als Kugelkopf ausgebildeten Implantatkopf aufweisen, an dem eine Tulpe zur Aufnahme und Klemmung eines Fixierungsstabs positionierbar angeordnet oder anordbar sein kann. Des Weiteren kann sie eine Werkzeugaufnahme für ein Schraubwerkzeug aufweisen, vorzugsweise innerhalb des Kugelkopfs, wobei die proximale Einlassöffnung in einer distalen Begrenzungsfläche der Werkzeugaufnahme ausgebildet ist.

Es ist von besonderem Vorteil, wenn die Werkzeugaufnahme des Knochenimplantats eine Verbindungsstruktur zum lösbaren Verbinden mit einer Injektionskanüle zum Injizieren von Knochenverbindungsmaterial aufweist. In ähnlicher Weise kann die Injektionskanüle eine Verbindungsstruktur zum lösbaren Verbinden mit einer Werkzeugaufnahme des Knochenimplantats aufweisen, insbesondere in Form eines Gewindes oder einer Bajonettstruktur. So kann das Instrument sicher derart am Implantat angeordnet und fixiert werden, dass ein Austreten von Knochenverbindungsmaterial über die Nahtstelle zwischen Implantat und Instrument sicher vermieden werden kann. Die Verbindungsstruktur kann insbesondere in Form eines Gewindes oder einer Bajonettstruktur ausgebildet sein. Dies ist einfach herstellbar und nutzerfreundlich handhabbar. Alternativ oder zusätzlich kann die Werkzeugaufnahme eine Eingriffkontur für ein Implantationswerkzeug aufweisen, mit dem das Knochenimplantat bei einer Implantation implantiert wird, insbesondere in Form eines Innensechskants oder einer Torx-Aufnahme. Die Injektionskanüle kann als Implantatwerkzeug ausgebildet sein und eine zur Werkzeugaufnahme passende Werkzeugkontur aufweisen, insbesondere in Form eines Außenseckskants oder eines Torx-Kopfes.

Zusammenfassend kann man sagen, dass durch die Erfindung eine Bildung von Zementstegen beim Abziehen einer Zementkanüle aus dem Implantat oder Knochenschraube sicher vermieden werden kann. Es kann eine Sollbruchstelle im in dem Kanal befindlichen Zement ausgebildet werden. Diese Bruchstelle liegt vorzugsweise unterhalb der Zementkanüle und maximal 10 mm unterhalb des Kugelkopfes der Knochenschraube. Die jeweiligen Hinterschnitte sind dabei so ausgebildet, dass eine Scherfläche gebildet wird, die gleich oder größer als die kleinste Querschnittsfläche des jeweiligen Kanals sowohl der Knochenschraube als auch der Zementkanüle ist.

Weitere Merkmale und Vorteile der vorliegenden Erfindung ergeben sich aus der folgenden beispielhaften und nicht beschränkenden Beschreibung der Erfindung anhand eines Pedikelschraubensystems als Beispiel für ein Knochenschraubensystem anhand von Figuren. Diese sind lediglich schematischer Natur und dienen nur dem Verständnis der Erfindung. Dabei zeigen:
Fig. 1 in einer Schnittansicht entlang der Längsachsen A und B ausschnittsweise ein Implantat nach einer ersten Ausführungsform der Erfindung in Form einer Pedikelschraube mit einem Implantationsinstrument nach einer ersten Ausführungsform der Erfindung in Wirkeingriff,
Fig. 2 eine Ausführungsform ähnlich der Fig. 1 in einer perspektivischen Ansicht,
Fig. 3 in einer Schnittansicht entlang der Längsachsen A und B ausschnittsweise ein Implantat nach einer zweiten Ausführungsform der Erfindung in Form einer Pedikelschraube mit einem Implantationsinstrument nach einer zweiten Ausführungsform der Erfindung in Wirkeingriff,
Fig. 4 eine Ausführungsform der Fig. 3 in einer perspektivischen Ansicht und
Fig. 5 die Ausführungsform der Figuren 1 und 2 mit nicht geschnittenen Implantationsinstrument.

Die vorliegende Beschreibung der Erfindung anhand der Figuren erfolgt mit Bezug auf eine Pedikelschraube 1. Die Erfindung betrifft jedoch insbesondere eine Knochenschraube. Die Bezeichnung Pedikelschraube ist daher als auf eine Knochenschraube gerichtet zu verstehen und umgekehrt.

Die Pedikelschraube 1 besitzt eine Längsachse A und weist einen in deren axialer Richtung verlaufenden Schraubenschaft 2 mit Außengewinde 3 oder Knochengewinde 3 auf. Am proximalen Ende des Schraubenschafts 2 ist ein kugelförmiger Schraubenkopf 4 ausgebildet, der wiederum eine Aufnahmehülse 5, auch als Tulpe 5 bezeichnet, infolge seiner Kugelform schwenkpositionierbar trägt. Die Tulpe 5 ist im Wesentlichen U-förmig ausgebildet mit gegenüberliegenden Wandabschnitten 6, 7, allgemein auch als Hülsenflanken bezeichnet, und einem dazwischen ausgebildeten, in radialer Richtung verlaufenden Spalt 8, der einen Aufnahmeraum für einen in den Figuren nicht dargestellten Längsträger oder Steg bildet. Die Tulpe 5 ist mit einem in axialer Richtung verlaufende Innengewinde 9 versehen, mit dem eine nicht gezeigte Klemmschraube (Setscrew) nach Einlegen des Längsträgers in die Tulpe 5 verschraubt wird, um diesen zu verklemmen und zu fixieren.

Im Schraubenschaft 2 der Pedikelschraube 1 ist ein Injektionskanal 10 ausgebildet, der in Richtung der Längsachse A ausgerichtet ist und (in den Figuren nicht zu erkennen) im Wesentlichen durch die gesamte Länge des Schraubenschafts 2 läuft. Der Injektionskanal 10 weist am kopfseitigen Ende eine proximale Einlassöffnung 11 und am distalen Ende des Schafts 2 und/oder über den Schraubenschaft 2 verteilt eine oder mehrere Auslassöffnungen auf. Durch den Injektionskanal 10 wird mittels des in den Figuren abschnittweise dargestellten Implantationsinstruments 12 Knochenverbindungsmaterial oder Knochenzement zu den Auslassöffnungen der in einem Knochen platzierten/eingeschraubten Pedikelschraube 1 gefördert, tritt aus diesen aus und zementiert die Pedikelschraube 1 im Knochen ein.

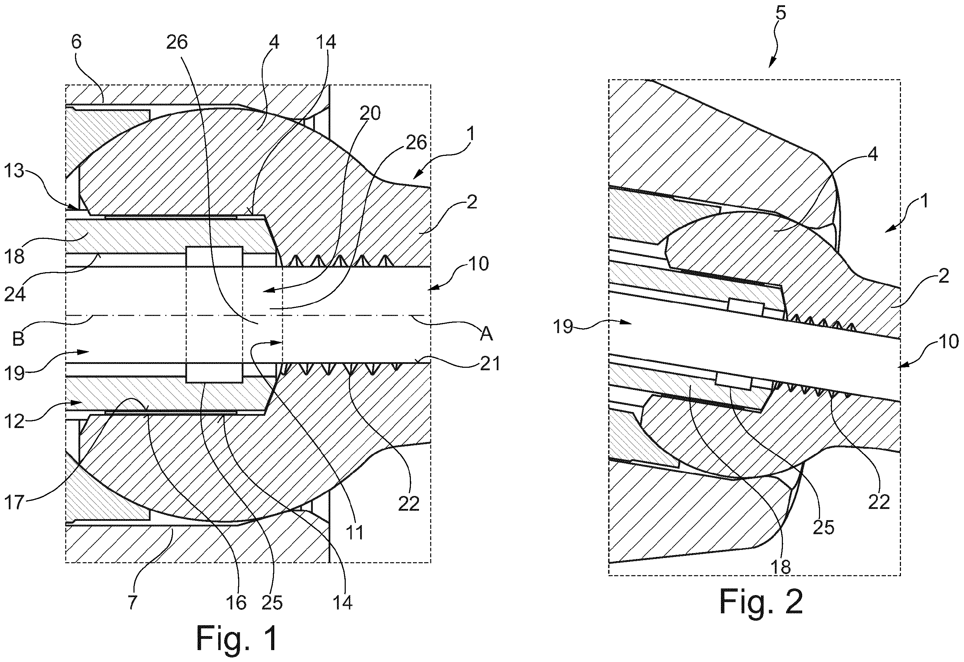

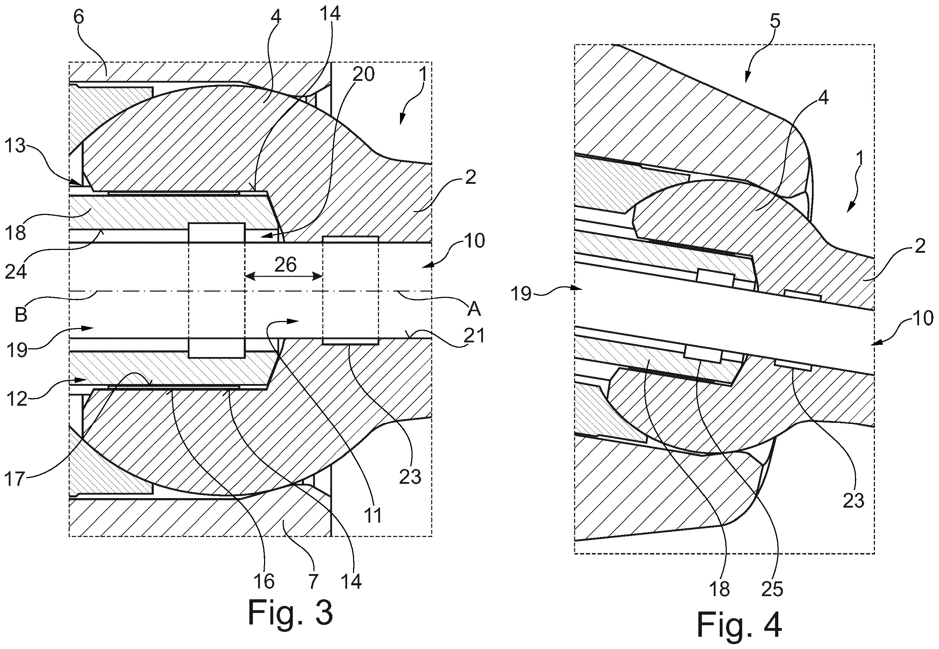

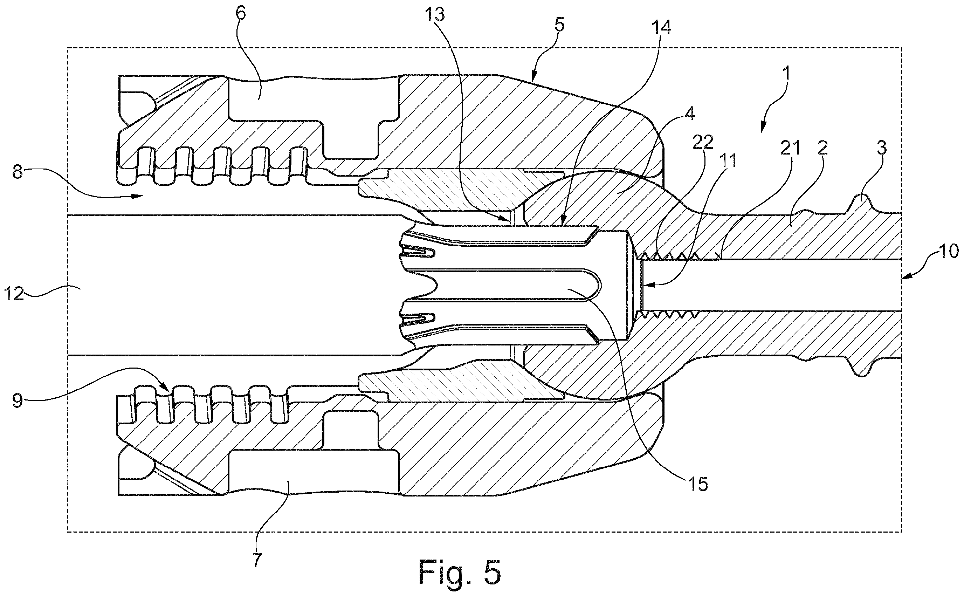

Der kugelförmige Schraubenkopf 4 weist eine zentrale Werkzeugaufnahme 13 in Form einer Ausnehmung auf. Die Werkzeugaufnahme 13 besitzt, wie insbesondere Figur 5 deutlich zeigt, eine als Torx-Aufnahme ausgebildete Eingriffkontur 14 für eine entsprechend geformte Werkzeugkontur 15 des Implantationsinstruments 12. Wie in den Figuren 1 und 3 gezeigt ist, ist darüber hinaus in der Werkzeugaufnahme 13 eine Verbindungskontur 16, hier in Form eines Innengewindes 16 ausgebildet, die sich mit der Eingriffkontur 14 überlagert. Das Innengewinde 16 dient einem schraubenden Eingriff mit einer Verbindungsstruktur 17 des Implantationsinstruments 12, hier in Form eines passend geformten Außengewindes 17.

Die Figuren 1 und 3 zeigen, dass das Implantationsinstrument 12 eine Injektionskanüle 18 mit einer Längsachse B aufweist. In dieser ist in Richtung der Längsachse B ein Innenkanal 19 ausgebildet, der am distalen Ende der Injektionskanüle 18 eine distale Auslassöffnung 20 aufweist. Das gegenüberliegende, in den Figuren nicht dargestellte Ende des Innenkanals 19 ist fluidisch mit einem Reservoir oder Behälter für Knochenverbindungsmaterial verbunden. Der Innenkanal 19 dient dazu, Knochenverbindungsmaterial von diesem Reservoir zur Auslassöffnung 20, und aus dieser über die proximale Einlassöffnung 11 in den Injektionskanal 10 der Pedikelschraube 1 zu fördern.

Zu diesem Zweck ist das Implantationsinstrument 12 in der nachfolgend beschriebenen Weise mit der Pedikelschraube 1 koppelbar: Über das Innengewinde 16 und das Außengewinde 17 wird das Implantationswerkzeug 12 derart in der Werkzeugaufnahme 13 des Schraubenkopfs 4 platziert und daran gehalten, dass über den Innenkanal 19 und den Injektionskanal 10 eine nach außen für das Knochenverbindungsmaterial dichte Strömungsbahn vom Zementreservoir zu den nicht gezeigten Auslassöffnungen der Pedikelschraube 1 ausgebildet ist. Bei einer derartigen bestimmungsgemäßer Anordnung des Instruments 12 in der Werkzeugaufnahme 13 stehen auch die Eingriffkontur 14 der Pedikelschraube 1 mit der Werkzeugkontur 15 des Implantationsinstruments 12 in Eingriff, derart, dass die Pedikelschraube 1 mit dem Implantationsinstrument 12 in einen Knochen eingeschraubt werden kann. Nach Erreichen der bestimmungsgemäßen Endlage der Pedikelschraube 1 wird über die Injektionskanüle Knochenverbindungsmaterial eingeleitet. Dieses strömt durch den Innenkanal 19 und den Injektionskanal 10 zu den Auslassöffnungen der Pedikelschraube 1 und aus diesen hinaus in zwischen der Pedikelschraube 1 und der Knochensubstanz vorliegende Kavitäten. Nach einer gewissen Zeitspanne ist das Material zumindest teilweise ausgehärtet und verfestigt, so dass das Implantationsinstrument 12 von der Pedikelschraube 1 gelöst und entfernt werden kann.

Zu diesem Zweck werden einfach das Innengewinde 16 und das Außengewinde 17 auseinander geschraubt, wodurch das Instrument 12 und dessen Injektionskanüle 18 in axialer Richtung vom Schraubenkopf entfernt werden. Dabei kommt die erfindungsgemäße Ausgestaltung der Kanäle 10, 19 mit einem Versatz zum Tragen. Im Injektionskanal 10 ist bei der Ausführungsform der Figuren 1 und 2 in einem an die proximale Einlassöffnung 11 angrenzenden Bereich in die Wand 21 ein Innengewinde 22 eingebracht, das den Versatz bzw. eine Mehrzahl von Versätzen ausbildet. In den Ausführungsformen der Figuren 3 und 4 ist in die Wand 21 des Injektionskanals 10 ein Hinterschnitt 23 eingebracht. Die Figuren 1 bis 4 zeigen, dass in die Wand 24 des Innenkanals 19 der Injektionskanüle 18 ebenfalls ein Versatz eingebracht ist, hier jeweils in Form eines Hinterschnitts 25. Eine Alternative ist hier ein in den Figuren nicht dargestelltes Innengewinde.

In den Kanälen 10, 19 vorliegendes Knochenverbindungsmaterial, siehe zum Beispiel in Figur 5, dringt im Rahmen des Injektionsvorgangs in die Gewindegänge des Innengewindes 22 bzw. die Hinterschnitte 23, 25 ein und härtet dort aus. Man kann also sagen, dass der Versatz der Kanäle 10, 19 in Form des Innengewindes 22 bzw. der Hinterschnitte 23, 25 in dem Knochenverbindungsmaterial abgeformt wird und dieses in dieser Form aushärtet. Es ist durch Formschluss mit dem entsprechenden Versatz nicht in Richtung der jeweiligen Längsachse A bzw. B positionierbar. Bei einem Entfernen des Implantationsinstruments 12 in axialer Richtung fort von der Pedikelschraube 1 kommt es daher zu einem Bruch bzw. Abscheren des innerhalb der Kanäle 10, 19 vorliegenden Knochenverbindungsmaterials in einem zwischen den beiden Hinterschnitten 23 und 25 bzw. zwischen dem Hinterschnitt 25 und dem Innengewinde gelegenen (Sollbruch-)Bereich 26. Dadurch wird sichergestellt, dass ausgehärtetes Knochenverbindungsmaterial möglichst nahe an der proximalen Einlassöffnung 11 des Kanals 10 in der Pedikelschraube 1 bzw. der Auslassöffnung 20 des Kanals 19 in der Injektionskanüle 18 abbricht und nicht übermäßig vorsteht.

In obiger Ausführungsform ist die Injektionskanüle 18 Teil des Implantationswerkzeugs 12. Bei der Injektionskanüle 18 kann es sich auch um ein separates, in die Pedikelschraube 1 einschraubbares Bauteil handeln, welches ebenfalls mit einem Hinterschnitt 25 bzw. radialen Versatz versehen ist.

### Bezugszeichenliste

- 1: Pedikelschraube
- 2: Schraubenschaft
- 3: Außengewinde, Knochengewinde
- 4: Schraubenkopf
- 5: Aufnahmehülse, Tulpe
- 6: Wandabschnitt
- 7: Wandabschnitt
- 8: Spalt, Aufnahmeraum
- 9: Innengewinde der Tulpe
- 10: Injektionskanal
- 11: proximale Einlassöffnung
- 12: Implantationsinstrument
- 13: Werkzeugaufnahme
- 14: Eingriffkontur
- 15: Werkzeugkontur, Torx-Kopf
- 16: Verbindungskontur, Innengewinde
- 17: Verbindungsstruktur, Außengewinde
- 18: Injektionskanüle
- 19: Innenkanal
- 20: distale Auslassöffnung
- 21: Wand
- 22: Innengewinde als Versatz
- 23: Hinterschnitt als Versatz
- 24: Wand
- 25: Hinterschnitt als Versatz
- 26: Bruchstelle

## Patentansprüche

1. Implantationsinstrument (12) für ein Knochenimplantat (1) vorzugsweise in Form einer Pedikelschraube, aufweisend eine Injektionskanüle (18) mit einem Innenkanal (19) mit einer Längsachse B zum Injizieren von Knochenverbindungsmaterial, wobei der Innenkanal (19) eine distale Auslassöffnung (20) für Knochenverbindungsmaterial aufweist und dort einen vorbestimmten Innenkanaldurchmesser hat, wobei die den Innenkanal (19) umgebende Wand (24) der Injektionskanüle (18) ausgehend von der distalen Auslassöffnung (20) einen zur Längsachse B radialen Versatz (25) aufweist, vorzugsweise ein in die Wand (24) des Innenkanals (19) eingebrachtes Gewinde oder eine in die Wand (24) des Innenkanals (19) eingebrachte Nut, aufweist, der sich gegenüber dem sowohl distal als auch proximal zum Versatz (25) ausgebildeten vorbestimmten Innenkanaldurchmesser radial nach außen erstreckt,
**dadurch gekennzeichnet, dass** der radiale Versatz (25) zumindest in einem Bereich der Wand (24) ausgebildet ist, der von der distalen Auslassöffnung (20) zwischen ca. 0,5 mm bis ca. 10 mm beabstandet ist.

2. Implantationsinstrument (12) nach Anspruch 1, **dadurch gekennzeichnet, dass** der radiale Versatz (25) der Injektionskanüle (18) durch zumindest eine in die Wand (24) des Innenkanals (19) eingebrachte Stufe oder Schulter ausgebildet ist und/oder in umfänglicher Richtung zumindest abschnittsweise umlaufend ausgebildet ist.

3. Implantationsinstrument (12) nach einem der vorhergehenden Ansprüche, **dadurch gekennzeichnet, dass** der radiale Versatz (25) der Injektionskanüle (18) durch einen in die Wand (24) des Innenkanals (19) eingebrachten Hinterschnitt (25) ausgebildet ist.

4. Implantationsinstrument (12) nach einem der vorhergehenden Ansprüche, **dadurch gekennzeichnet, dass** der radiale Versatz (25) der Injektionskanüle (18) in einem Bereich der Wand (24) ausgebildet ist, der von der distalen Auslassöffnung (20) zwischen ca. 1 mm bis ca. 7,5 mm, bevorzugt zwischen ca. 1,5 mm bis ca. 5 mm und noch bevorzugter zwischen ca. 2 mm bis ca. 3 mm beabstandet ist.

5. Implantationsinstrument (12) nach einem der vorhergehenden Ansprüche, **dadurch gekennzeichnet, dass** die Injektionskanüle (18) eine Verbindungsstruktur (17) zum lösbaren Verbinden mit einer Werkzeugaufnahme (13) des Knochenimplantats aufweist, insbesondere in Form eines Gewindes (17) oder einer Bajonettstruktur.

6. Implantationsinstrument (12) nach einem der vorhergehenden Ansprüche, **dadurch gekennzeichnet, dass** die Injektionskanüle (18) als Implantatwerkzeug ausgebildet ist und einen zur Werkzeugaufnahme (13) passende Werkzeugkontur (15) aufweist, insbesondere in Form eines Außensechskants oder eines Torx-Kopfes (15).

7. Implantationssystem mit
einem Implantationsinstrument (12) für ein Knochenimplantat (1) vorzugsweise in Form einer Pedikelschraube, aufweisend eine Injektionskanüle (18) mit einem Innenkanal (19) mit einer Längsachse B zum Injizieren von Knochenverbindungsmaterial, wobei der Innenkanal (19) eine distale Auslassöffnung (20) für Knochenverbindungsmaterial aufweist und dort einen vorbestimmten Innenkanaldurchmesser hat, wobei die den Innenkanal (19) umgebende Wand (24) der Injektionskanüle (18) ausgehend von der distalen Auslassöffnung (20) einen zur Längsachse B radialen Versatz (25) aufweist, vorzugsweise ein in die Wand (24) des Innenkanals (19) eingebrachtes Gewinde oder eine in die Wand (24) des Innenkanals (19) eingebrachte Nut aufweist, der sich gegenüber dem vorbestimmten Innenkanaldurchmesser, der sich sowohl distal als auch proximal zum Versatz (25) ausbildet, radial nach außen erstreckt, vorzugsweise einem Implantationsinstrument (12) nach einem der vorhergehenden Ansprüche, sowie
einem Knochenimplantat (1) mit einem distalen Implantatschaft (2), der zum Versenken in einem Knochen ausgebildet ist, und einem proximalen Implantatkopf (4) mit einer Werkzeugaufnahme (13) für ein Implantationswerkzeug (12), von der sich ein Injektionskanal (10) zum Injizieren eines Knochenverbindungsmaterials in den Implantatschaft (2) erstreckt und dort einen vorbestimmten Injektionskanaldurchmesser hat, wobei die den Injektionskanal (10) umgebende Wand (21) ausgehend von der Werkzeugaufnahme (13) einen Versatz, vorzugsweise ein in die Wand (21) des Injektionskanals (10) eingebrachtes Gewinde (22) oder eine in die Wand (21) des Injektionskanals (10) eingebrachte Nut (23), aufweist, der sich gegenüber dem vorbestimmten Injektionskanaldurchmesser proximal und distal zum Versatz radial nach außen erstreckt.

8. Implantationssystem nach Anspruch 7, **dadurch gekennzeichnet, dass** der radiale Versatz (22, 23) des Knochenimplantats (1) durch zumindest eine in der Wand (21) des Injektionskanals (10) eingebrachte Stufe oder Schulter ausgebildet ist und/oder in Umfangsrichtung zumindest abschnittsweise umlaufend ausgebildet ist.

9. Implantationssystem nach einem der Ansprüche 7 und 8, **dadurch gekennzeichnet, dass** der radiale Versatz (22, 23) des Knochenimplantats (1) durch einen in die Wand (21) des Injektionskanals (10) eingebrachten Hinterschnitt (23) ausgebildet ist.

10. Implantationssystem nach einem der Ansprüche 7 bis 9, **dadurch gekennzeichnet, dass** der radiale Versatz (22, 23) des Knochenimplantats (1) in einem Bereich der Wand (21) ausgebildet ist, der von der proximalen Einlassöffnung (11) wenigstens ca. 0,5 mm und maximal ca. 10 mm beabstandet ist, vorzugsweise zwischen ca. 1 mm bis ca. 7,5 mm, bevorzugter zwischen ca. 1,5 mm bis ca. 5 mm und noch bevorzugter zwischen ca. 2 mm bis ca. 3 mm beabstandet ist.

11. Implantationssystem nach einem der Ansprüche 7 bis 10, **dadurch gekennzeichnet, dass** die Tiefe des Versatzes (22, 23) des Knochenimplantats (1) ausgehend von dem Injektionskanaldurchmesser in radialer Richtung zwischen ca. 0,1 mm bis 3 mm, vorzugsweise zwischen ca. 0,2 mm bis 2 mm und besonders bevorzugt zwischen ca. 0,3 mm bis ca. 1,5 mm beträgt.

12. Implantationssystem nach einem der Ansprüche 7 bis 11, **dadurch gekennzeichnet, dass** das Knochenimplantat (1) eine Knochenschraube (1) ist, insbesondere eine Pedikelschraube (1), deren Implantatkopf (4) insbesondere als Kugelkopf (4) ausgebildet ist und/oder eine Werkzeugaufnahme (13) für ein Schraubwerkzeug (12) aufweist, wobei die proximale Einlassöffnung (11) in einer distalen Begrenzungsfläche der Werkzeugaufnahme (13) ausgebildet ist.

13. Implantationssystem nach einem der Ansprüche 7 bis 12, **dadurch gekennzeichnet, dass** die Werkzeugaufnahme (13) des Knochenimplantats (1) eine Verbindungsstruktur (16) zum lösbaren Verbinden mit einer Injektionskanüle (18) zum Injizieren von Knochenverbindungsmaterial aufweist, insbesondere in Form eines Gewindes, Innengewindes (16) oder einer Bajonettstruktur und/oder dass die Werkzeugaufnahme (13) eine Eingriffkontur (14) für ein Implantationswerkzeug (12) aufweist, mit dem das Knochenimplantat (1) bei einer Implantation implantiert wird, insbesondere in Form eines Innensechskants oder einer Torx-Aufnahme.

14. Knochenimplantat (1) in Form einer Pedikelschraube für ein Implantationssystem nach einem der Ansprüche 7 bis 13 mit einem distalen Implantatschaft (2), der zum Versenken in einem Knochen ausgebildet ist, und einem proximalen Implantatkopf (4) mit einer Werkzeugaufnahme (13) für ein Implantationswerkzeug (12), von der sich ein Injektionskanal (10) zum Injizieren eines Knochenverbindungsmaterials in den Implantatschaft (2) erstreckt und dort einen vorbestimmten Injektionskanaldurchmesser hat,
**dadurch gekennzeichnet, dass**
die den Injektionskanal (10) umgebende Wand (21) ausgehend von der Werkzeugaufnahme (13) einen Versatz in Form einer in die Wand (21) des Injektionskanals (10) eingebrachten singulären Nut (23) aufweist, die sich gegenüber dem vorbestimmten Injektionskanaldurchmesser, der sich proximal und distal zum Versatz ausbildet, radial nach außen erstreckt, wobei die singuläre Nut (23) durch einen einzigen, in die Wand (21) des Injektionskanals (10) eingebrachten Hinterschnitt ausgebildet ist.

15. Knochenimplantat (1) nach Anspruch 14, **dadurch gekennzeichnet, dass** die Nut (23) in einem Bereich der Wand (21) ausgebildet ist, der von der proximalen Einlassöffnung (11) wenigstens ca. 0,5 mm und maximal ca. 10 mm beabstandet ist, vorzugsweise zwischen ca. 1 mm bis ca. 7,5 mm, bevorzugter zwischen ca. 1,5 mm bis ca. 5 mm und noch bevorzugter zwischen ca. 2 mm bis ca. 3 mm in Richtung Distal beabstandet ist.

16. Knochenimplantat (1) nach einem der 14 und 15, **dadurch gekennzeichnet, dass** die Tiefe der Nut (23) in radialer Richtung ausgehend von dem Injektionskanaldurchmesser zwischen ca. 0,1 mm bis 3 mm, vorzugsweise zwischen ca. 0,2 mm bis 2 mm und besonders bevorzugt zwischen ca. 0,3 mm bis ca. 1,5 mm beträgt.

## Claims

1. An implantation instrument (12) for a bone implant (1) preferably in the form of a pedicle screw, the implantation instrument (12) comprising an injection cannula (18) including an inner channel (19) having a longitudinal axis B for injection of bone connection material, wherein the inner channel (19) comprises a distal outlet opening (20) for bone connection material and has a predetermined inner channel diameter, wherein the wall (24) of the injection cannula (18) surrounding the inner channel (19) has a radial offset (25) with respect to the longitudinal axis B, preferably having a thread inserted in the wall (24) of the inner channel (19) or a groove (25) inserted into the wall (24) of the inner channel (19), the radial offset (25) extending radially outward with respect to the predetermined inner channel diameter, the predetermined inner channel diameter being formed both proximally and distally relative to the radial offset (25).

2. The implantation instrument (12) according to claim 1, **characterized in that** the radial offset (25) of the injection cannula (18) is formed by at least one step or shoulder machined in the wall (24) of the inner channel (19) and/or is formed so as to circumferentially extend in the circumferential direction at least in sections.

3. The implantation instrument (12) according to one of the preceding claims, **characterized in that** the radial offset (25) of the injection cannula (18) is formed by an undercut (25) machined in the wall (24) of the inner channel (19).

4. The implantation instrument (12) according to one of the preceding claims, **characterized in that** the radial offset (25) of the injection cannula (18) is formed in an area of the wall (24), wherein the area is spaced from the distal outlet opening (20) at least by about 1 mm to about 7.5 mm, preferably between about 1.5 mm to about 5 mm and more preferably between about 2 mm to about 3 mm.

5. The implantation instrument (12) according to one of the preceding claims, **characterized in that** the injection cannula (18) comprises a connection structure (17) for a detachable connection with the tool receiver (13) of the bone implant (1), in particular in the form of a thread (17) or a bayonet structure.

6. The implantation instrument (12) according to one of the preceding claims, **characterized in that** the injection cannula (18) is designed as an implantation tool (12) and comprises a tool contour (15) matched with the tool receiver (13), in particular in the form of an external hexagon or a Torx head (15).

7. Implantation system with
an implantation instrument (12) for a bone implant (1) preferably in the form of a pedicle screw, comprising an injection cannula (18) with an inner channel (19) having a longitudinal axis B for injecting bone connection material, wherein the inner channel (19) has a distal outlet opening (20) for bone connection material and has a predetermined inner channel diameter there, wherein the wall (24) of the injection cannula (18) surrounding the inner channel (19) has a radial offset (25) relative to the longitudinal axis B starting from the distal outlet opening (20), preferably has a thread machined into the wall (24) of the inner channel (19) or a groove machined into the wall (24) of the inner channel (19), wherein the offset extends radially outward relative to the predetermined inner channel diameter, which is formed both distally and proximally to the offset (25), preferably with an implantation instrument (12) according to one of the preceding claims, and
a bone implant (1) with a distal implant shaft (2) which is designed for embedding in a bone, and a proximal implant head (4) with a tool receiver (13) for an implantation tool (12), wherein an injection channel (10) for injecting a bone bonding material extends from the tool receiver (13) into the implant shaft (2) and has a predetermined injection channel diameter there, wherein the wall (21) surrounding the injection channel (10), starting from the tool receiver (13), has an offset, preferably a thread (22) machined into the wall (21) of the injection channel (10) or a groove (23) machined into the wall (21) of the injection channel (10), which extends radially outward relative to the predetermined injection channel diameter proximally and distally to the offset.

8. The implantation system according to claim 7, **characterized in that** the radial offset (22, 23) of the bone implant (1) is formed by at least one step or shoulder machined in the wall (21) of the injection channel (10) and/or is formed so as to circumferentially extend in a circumferential direction at least in sections.

9. The implantation system according to one of the claims 7 and 8, **characterized in that** the radial offset (22, 23) of the bone implant (1) is formed by an undercut (23) machined in the wall (21) of the injection channel (10).

10. The implantation system according to one of the claims 7 to 9, **characterized in that** the radial offset (22, 23) of the bone implant (1) is formed in an area of the wall (21), wherein the area is spaced from the proximal inlet opening (11) at least by about 0.5 mm and at most about 10 mm, is preferably spaced between about 1 mm and about 7.5 mm, more preferably between about 1.5 mm and about 5 mm and even more preferably between about 2 mm and about 3 mm.

11. The implantation system according to one of the claims 7 to 10, **characterized in that** the depth of the offset (22, 23) of the bone implant (1) starting from the injection channel diameter in a radial direction amounts to between about 0.1 mm to 3 mm, is preferably between about 0.2 mm to 2 mm and particularly preferably between about 0.3 mm to about 1.5 mm.

12. The implantation system according to one of the claims 7 to 11, **characterized in that** the bone implant (1) is a bone screw (1), in particular a pedicle screw (1), the implant head (4) of which is formed in particular as a ball head (4), and/or comprises a tool receiver (13) for a screw-driving tool (12), wherein the proximal inlet opening (11) is formed in a distal boundary surface of the tool receiver (13).

13. The implantation system according to one of the claims 7 to 12, **characterized in that** the tool receiver (13) of the bone implant (1) comprises a connection structure (16) for a detachable connection with an injection cannula (18) for injection of bone connection material, in particular in the form of a thread, an inner thread (16) or a bayonet structure, and/or that the tool receiver (13) comprises an engagement contour (14) for an implantation tool (12) by which the bone implant (1) is implanted during an implantation, in particular in the form of a hexagon socket or a Torx socket.

14. A bone implant (1) in the form of a pedicle screw for an implantation system according to one of the claims 7 to 13, the bone implant (1) comprising a distal implant shank (2) formed for being embedded in a bone, and a proximal implant head (4) having a tool receiver (13) for the implantation tool (12), wherein from the tool receiver (13), an injection channel (10) for injecting a bone connection material extends into the implant shank (2) and forms a predetermined injection channel diameter there,
**characterized in that**
the wall (21) surrounding the injection channel (10) has, starting from the tool receiver (13), an offset in the form of a singular groove (23) which is formed in the wall (21) of the injection channel (10) and which extends radially outward with respect to the predetermined injection channel diameter which is formed proximal and distal to the offset, the singular groove (23) being formed by a single undercut formed in the wall (21) of the injection channel (10).

15. The bone implant (1) according to claim 14, **characterized in that** the groove (23) is formed in a region of the wall (21) which is spaced from the proximal inlet opening (11) by at least about 0.5 mm and at most about 10 mm, preferably between about 1 mm and about 7.5 mm, more preferably between about 1.5 mm and about 5 mm and even more preferably between about 2 mm and about 3 mm in the distal direction.

16. The bone implant (1) according to one of claims 14 and 15, **characterized in that** the depth of the groove (23) in the radial direction starting from the injection channel diameter is between about 0.1 mm to 3 mm, preferably between about 0.2 mm to 2 mm and particularly preferably between about 0.3 mm to about 1.5 mm.

## Revendications

1. Instrument d'implantation (12) pour un implant osseux (1), de préférence sous forme d'une vis pédiculaire, présentant une canule d'injection (18) avec un canal intérieur (19) avec un axe longitudinal B pour l'injection de matériau de liaison osseuse, dans lequel le canal intérieur (19) présente une ouverture de sortie distale (20) pour le matériau de liaison osseuse et y présente un diamètre de canal intérieur prédéterminé, dans lequel la paroi (24) de la canule d'injection (18) entourant le canal intérieur (19) présente, en partant de l'ouverture de sortie distale (20), un décalage radial (25) par rapport à l'axe longitudinal B, de préférence un filetage réalisé dans la paroi (24) du canal intérieur (19) ou une rainure réalisée dans la paroi (24) du canal intérieur (19), qui s'étend radialement vers l'extérieur par rapport au diamètre de canal intérieur prédéterminé formé aussi bien distalement que proximalement par rapport au décalage (25),
**caractérisé en ce que** le décalage radial (25) est formé au moins dans une zone de la paroi (24) qui est espacée de l'ouverture de sortie distale (20) d'environ 0,5 mm à environ 10 mm.

2. Instrument d'implantation (12) selon la revendication 1, **caractérisé en ce que** le décalage radial (25) de la canule d'injection (18) est formé par au moins une marche ou un épaulement réalisé dans la paroi (24) du canal intérieur (19) et/ou est formé de manière circonférentielle au moins par sections dans la direction circonférentielle.

3. Instrument d'implantation (12) selon l'une quelconque des revendications précédentes, **caractérisé en ce que** le décalage radial (25) de la canule d'injection (18) est formé par une contre-dépouille (25) réalisée dans la paroi (24) du canal intérieur (19).

4. Instrument d'implantation (12) selon l'une quelconque des revendications précédentes, **caractérisé en ce que** le décalage radial (25) de la canule d'injection (18) est formé dans une zone de la paroi (24), qui est espacée de l'ouverture de sortie distale (20) d'entre environ 1 mm et environ 7,5 mm, de préférence entre environ 1,5 mm et environ 5 mm, et encore plus préférentiellement entre environ 2 mm et environ 3 mm.

5. Instrument d'implantation (12) selon l'une quelconque des revendications précédentes, **caractérisé en ce que** la canule d'injection (18) présente une structure de liaison (17) pour la liaison amovible à un logement d'outil (13) de l'implant osseux, en particulier sous forme d'un filetage (17) ou d'une structure à baïonnette.

6. Instrument d'implantation (12) selon l'une quelconque des revendications précédentes, **caractérisé en ce que** la canule d'injection (18) est formée en tant qu'outil d'implantation et présente un contour d'outil (15) adapté au logement d'outil (13), en particulier sous forme d'un six pans extérieur ou d'une tête Torx (15).

7. Système d'implantation avec
un instrument d'implantation (12) pour un implant osseux (1), de préférence sous forme d'une vis pédiculaire, présentant une canule d'injection (18) avec un canal intérieur (19) avec un axe longitudinal B pour l'injection de matériau de liaison osseuse, dans lequel le canal intérieur (19) présente une ouverture de sortie distale (20) pour le matériau de liaison osseuse et y présente un diamètre de canal intérieur prédéterminé, dans lequel la paroi (24) de la canule d'injection (18) entourant le canal intérieur (19) présente, en partant de l'ouverture de sortie distale (20), un décalage radial (25) par rapport à l'axe longitudinal B, de préférence un filetage réalisé dans la paroi (24) du canal intérieur (19) ou une rainure réalisée dans la paroi (24) du canal intérieur (19), qui s'étend radialement vers l'extérieur par rapport au diamètre prédéterminé du canal intérieur, qui se forme aussi bien distalement que proximalement par rapport au décalage (25), de préférence un instrument d'implantation (12) selon l'une quelconque des revendications précédentes, ainsi que
un implant osseux (1) avec une tige d'implant distale (2), qui est formée pour s'enfoncer dans un os, et une tête d'implant proximale (4) avec un logement d'outil (13) pour un outil d'implantation (12), à partir duquel un canal d'injection (10) s'étend pour l'injection d'un matériau de liaison osseuse dans la tige d'implant (2) et y présente un diamètre de canal d'injection prédéterminé, dans lequel la paroi (21) entourant le canal d'injection (10) présente, à partir du logement d'outil (13), un décalage, de préférence un filetage (22) réalisé dans la paroi (21) du canal d'injection (10) ou une rainure (23) réalisée dans la paroi (21) du canal d'injection (10), qui s'étend radialement vers l'extérieur par rapport au diamètre de canal d'injection prédéterminé, proximalement et distalement par rapport au décalage.

8. Système d'implantation selon la revendication 7, **caractérisé en ce que** le décalage radial (22, 23) de l'implant osseux (1) est formé par au moins une marche ou un épaulement réalisé dans la paroi (21) du canal d'injection (10) et/ou est formé de manière circonférentielle au moins par sections dans la direction circonférentielle.

9. Système d'implantation selon l'une quelconque des revendications 7 et 8, **caractérisé en ce que** le décalage radial (22, 23) de l'implant osseux (1) est formé par une contre-dépouille (23) réalisée dans la paroi (21) du canal d'injection (10).

10. Système d'implantation selon l'une quelconque des revendications 7 à 9, **caractérisé en ce que** le décalage radial (22, 23) de l'implant osseux (1) est formé dans une zone de la paroi (21) qui est espacée de l'ouverture d'entrée proximale (11) d'au moins environ 0,5 mm et d'au plus environ 10 mm, de préférence entre environ 1 mm et environ 7,5 mm, plus préférentiellement entre environ 1,5 mm et environ 5 mm et encore plus préférentiellement entre environ 2 mm et environ 3 mm.

11. Système d'implantation selon l'une quelconque des revendications 7 à 10, **caractérisé en ce que** la profondeur du décalage (22, 23) de l'implant osseux (1) à partir du diamètre de canal d'injection en direction radiale est entre environ 0,1 mm et 3 mm, de préférence entre environ 0,2 mm et 2 mm et le plus préférentiellement entre environ 0,3 mm et environ 1,5 mm.

12. Système d'implantation selon l'une quelconque des revendications 7 à 11, **caractérisé en ce que** l'implant osseux (1) est une vis à os (1), en particulier une vis pédiculaire (1), dont la tête d'implant (4) est formée en particulier en tant que rotule (4) et/ou présente un logement d'outil (13) pour un outil de vissage (12), dans lequel l'ouverture d'entrée proximale (11) est formée dans une surface de délimitation distale du logement d'outil (13).

13. Système d'implantation selon l'une quelconque des revendications 7 à 12, **caractérisé en ce que** le logement d'outil (13) de l'implant osseux (1) présente une structure de liaison (16) pour la liaison amovible avec une canule d'injection (18) pour l'injection d'un matériau de liaison osseuse, en particulier sous forme d'un filetage, filetage intérieur (16) ou d'une structure à baïonnette et/ou **en ce que** le logement d'outil (13) présente un contour d'engagement (14) pour un outil d'implantation (12) avec lequel l'implant osseux (1) est implanté lors d'une implantation, en particulier sous forme d'un six pans intérieur ou d'un logement Torx.

14. Implant osseux (1) sous forme d'une vis pédiculaire pour un système d'implantation selon l'une quelconque des revendications 7 à 13, avec une tige d'implant distale (2) qui est formée pour s'enfoncer dans un os, et une tête d'implant proximale (4) avec un logement d'outil (13) pour un outil d'implantation (12), à partir duquel un canal d'injection (10) s'étend pour l'injection d'un matériau de liaison osseuse dans la tige d'implant (2) et y présente un diamètre de canal d'injection prédéterminé,
**caractérisé en ce que**
la paroi (21) entourant le canal d'injection (10) présente, en partant du logement d'outil (13), un décalage sous forme d'une rainure singulière (23) réalisée dans la paroi (21) du canal d'injection (10), qui s'étend radialement vers l'extérieur par rapport au diamètre de canal d'injection prédéterminé, qui se forme proximalement et distalement par rapport au décalage, dans lequel la rainure singulière (23) est formée par une seule contre-dépouille réalisée dans la paroi (21) du canal d'injection (10).

15. Implant osseux (1) selon la revendication 14, **caractérisé en ce que** la rainure (23) est formée dans une zone de la paroi (21) qui est espacée de l'ouverture d'entrée proximale (11) d'au moins environ 0,5 mm et d'au plus environ 10 mm, de préférence entre environ 1 mm et environ 7,5 mm, plus préférentiellement entre environ 1,5 mm et environ 5 mm et encore plus préférentiellement entre environ 2 mm et environ 3 mm en direction distale.

16. Implant osseux (1) selon l'une quelconque des revendications 14 et 15, **caractérisé en ce que** la profondeur de la rainure (23) en direction radiale à partir du diamètre de canal d'injection est entre environ 0,1 mm et 3 mm, de préférence entre environ 0,2 mm et 2 mm et le plus préférentiellement entre environ 0,3 mm et environ 1,5 mm.
